# EUROPÄISCHE PATENTSCHRIFT

(11) **EP 3 429 516 B1**
(45) Veröffentlichungstag und Bekanntmachung des Hinweises auf die Patenterteilung: **29.04.2020**
(21) Anmeldenummer: 17711603.5
(22) Anmeldetag: 14.03.2017
(51) Int. Cl.: A61F 5/03, A61H 1/02, A61F 2/68, A61F 4/00

(54) **ORTHOPÄDIETECHNISCHE VORRICHTUNG**
ORTHOPEDIC DEVICE
DISPOSITIF ORTHOPÉDIQUE

(30) Priorität: 16.03.2016 DE 102016104880
(43) Veröffentlichungstag der Anmeldung: 23.01.2019
(73) Patentinhaber: Ottobock SE & Co. KGaA, 37115 Duderstadt (DE)
(72) Erfinder: KURZWEG, Annedore, 37085 Göttingen (DE); KROLL-ORYWAHL, Olaf, 37154 Northeim (DE); BORNMANN, Jonas, 37115 Duderstadt (DE)
(74) Vertreter: Gramm, Lins & Partner Patent- und Rechtsanwälte PartGmbB
(86) Internationale Anmeldenummer: PCT/EP2017/055888
(87) Internationale Veröffentlichungsnummer: WO 2017/157875

(56) Entgegenhaltungen:
- EP-A2- 2 724 825
- WO-A2-2012/099995
- US-A1- 2013 090 931
- US-A1- 2014 158 839

## Beschreibung

Die Erfindung betrifft eine orthopädietechnische Vorrichtung für wenigstens eine obere Extremität, wobei die Vorrichtung wenigstens ein Positionierelement aufweist, das wenigstens zwei Bewegungselemente aufweist, die relativ zueinander in eine erste Richtung bewegbar sind, wobei einer Bewegung der Bewegungselemente relativ zueinander in eine erste Richtung ein Widerstand entgegensteht.

Derartige Vorrichtungen sind aus der US 2014/158839 A1, der WO 2012/99995 A2 und der EP 2 724 825 A2 bekannt.

Eine orthopädietechnische Vorrichtung kann eine Prothese oder eine Orthese, insbesondere ein Exoskelett sein. Vorliegend ist sie für wenigstens eine obere Extremität ausgebildet, sodass sie wenigstens einen Teil eines oder beider Arme und/oder einer oder beider Schultern und/oder des Rückens unterstützt, stützt, schützt oder ersetzt. Eine solche orthopädietechnische Vorrichtung ist in vielen unterschiedlichen Ausgestaltungen aus dem Stand der Technik bekannt und verfügt über wenigstens ein Gelenk, durch das zwei Bauteile der orthopädietechnischen Vorrichtung relativ zueinander bewegbar aneinander angeordnet und miteinander verbunden werden können.

Oftmals werden durch orthopädietechnische Vorrichtungen Gelenke der oberen Extremität unterstützt, gestützt, geführt oder geschützt. Bei einer Ellenbogenorthese beispielsweise verbindet das Gelenk einen Oberarmanteil und einen Unterarmenteil der orthopädietechnischen Vorrichtung schwenkbar miteinander, sodass das Gelenk für die Orthese die Funktion eines Ellenbogengelenks übernimmt. Natürlich sind auch andere Gelenke für andere Orthesen und andere Körperteile der oberen Extremität aus dem Stand der Technik bekannt.

Aus der US 3,769,636 ist ein Rollstuhl bekannt, der insbesondere für Personen geeignet ist, die über vier gelähmte Gliedmaßen verfügen. Am Kopf wird eine Steuervorrichtung angeordnet, die über mehrere Kardan-Gelenke mit dem Rollstuhl verbunden ist. Auf diese Weise lassen sich Bewegungen des Kopfes erfassen, die anschließend verwendet werden können, um beispielsweise ein Armpolster oder eine Armauflage den Wünschen des Patienten entsprechend zu bewegen. Dabei wird der ansonsten vollständig immobile Arm, der auf der Armauflage aufliegt, über Aktoren und Motoren bewegt.

Eine ähnliche Steuervorrichtung, die beispielsweise durch Blasen oder Saugen, durch Beißdruck oder durch Bewegung des Kopfes gesteuert werden kann, ist aus der US 4,865,610 bekannt.

Aus der WO 2012/099995 A2 ist beispielsweise eine orthopädietechnische Vorrichtung bekannt, die Personen unterstützen soll, die viel mit ausgestreckten Armen, insbesondere über Kopf arbeiten müssen. Sie verfügt über Armablagestützen und Kompensationselemente, die insbesondere gespannte Federn aufweisen können und durch die permanent eine Kraft auf die Oberarme ausgeübt wird, die diese nach oben drückt. Dadurch wird das Arbeiten in der ansonsten sehr unbequemen Position erleichtert. Die von den Federelementen aufgebrachte Kraft dient dazu, das Eigengewicht der Arme zu kompensieren, sodass der Träger der orthopädietechnischen Einrichtung selbst die dafür benötigte Kraft nicht mit den Oberarmen aufbringen muss. Durch Veränderung der Spannung der verwendeten Federn lässt sich die aufgebrachte Kraft vor der Benutzung der orthopädietechnischen Einrichtung einstellen, um beispielsweise den Bedürfnissen unterschiedlicher Patienten, die beispielsweise durch unterschiedlich schwere Arme hervorgerufen werden, Rechnung zu tragen.

Eine ähnliche Vorrichtung ist in der US 2014/0158839 A1 offenbart.

Nachteilig ist jedoch, dass im Betrieb der Vorrichtung die aufgebrachte Kraft und damit ein Widerstand, der der Bewegung der Bewegungselemente des wenigstens einen Positionierelementes in der ersten Richtung entgegensteht, nicht eingestellt werden kann. Im aus dem Stand der Technik bekannten Beispiel wird durch die Kraft das Halten der Arme in erhobener Position erleichtert, das Absenken der Arme naturgemäß jedoch erschwert. Durch die aufgebrachte Kraft ist folglich ein Widerstand definiert, der einer Bewegung des Gelenkes entgegenwirkt, die dem Absenken der Arme entspricht. Dieser Widerstand ist bei dem aus dem Stand der Technik bekannten Beispiel nur in eine Bewegungsrichtung des Gelenkes vorhanden, kann jedoch bei anderen Ausführungsformen auch in beide oder mehrere Richtungen ausgeübt werden.

Der Erfindung liegt daher die Aufgabe zugrunde, eine orthopädietechnische Vorrichtung so weiterzuentwickeln, dass der Widerstand, der der Bewegung der Bewegungselemente des Positionierelementes in der ersten Richtung entgegensteht, möglichst intuitiv und einfach gesteuert und eingestellt werden kann.

Die Erfindung löst die gestellte Aufgabe durch eine orthopädietechnische Vorrichtung für wenigstens eine obere Extremität gemäß dem Oberbegriff des Anspruchs 1, die sich dadurch auszeichnet, dass die Vorrichtung eine Steuerung aufweist, die eingerichtet ist, den Widerstand in Abhängigkeit einer Position und/oder einer Lage des Kopfes des Trägers der Vorrichtung einzustellen.

Bei der erfindungsgemäßen orthopädietechnischen Vorrichtung wird folglich über die Position und/oder die Lage des Kopfes des Trägers der Vorrichtung eingestellt, wie groß der Widerstand ausgebildet sein soll, der einer Bewegung der Bewegungselemente des wenigstens einen Positionierelementes in der ersten Richtung entgegensteht. Dadurch wird eine intuitive Steuerung möglich, ohne dass dafür Hände oder Arme verwendet werden müssen. Handelt es sich bei der orthopädietechnischen Vorrichtung um eine Orthese, sind Hände und Arme gegebenenfalls motorisch eingeschränkt oder sollen vor übermäßigen Belastungen und extremen Bewegungen geschützt werden. Zusätzliche Bewegungen sind mit der erfindungsgemäßen orthopädietechnischen Vorrichtung für die Einstellung des Widerstandes nicht nötig. Handelt es sich bei der orthopädietechnischen Vorrichtung um eine Prothese sind gegebenenfalls Hände oder Teile der Arme nicht mehr in natürlicher Form erhalten, sodass auch in diesem Fall eine Steuerung über eine Einstellung durch manuelle Betätigung eines Betätigungselementes nicht oder nur schwer möglich wäre. Insbesondere für den Anwendungsfall einer unterstützenden orthopädietechnischen Vorrichtung, die beispielsweise Personen unterstützt, die über Kopf oder in unbequemen Positionen arbeiten müssen, ist die erfindungsgemäße orthopädietechnische Vorrichtung mit der Steuerung durch die Position und/oder Lage des Kopfes von Vorteil, da die Hände obwohl vorhanden und motorisch nicht beeinträchtigt nicht verwendet werden müssen, und so für andere Tätigkeiten zur Verfügung stehen.

Ein Positionierelement im Sinne der vorliegenden Erfindung ist beispielsweise ein Gelenk, bei dem die wenigstens zwei Bewegungselemente gegeneinander verschwenkbar sind, oder eine Verschiebeeinrichtung, die beispielsweise in der Länge veränderbar ist, sodass die beiden Bewegungselemente wie bei einer Teleskopstange gegeneinander verschiebbar sind.

Ein Positionierelement im Sinne der vorliegenden Erfindung kann auch ein Kugelgelenk sein. Im diesem Fall ist eine Verschwenkung der wenigstens zwei Bewegungselemente in jede Richtung, zumindest aber in nahezu jede Richtung, möglich. Beispielsweise über Dämpfungselemente oder Bremselemente kann ein Widerstand, der einer derartigen Verschwenkung der wenigstens zwei Bewegungselemente in eine Schwenkrichtung entgegensteht eingestellt werden. Dabei kann die vom Träger der orthopädietechnischen Einrichtung auf das Gelenk ausgeübte Kraft oder ein Moment gemessen oder erfasst werden und als Steuerparameter für den der jeweiligen Bewegung entgegenstehenden Widerstand verwendet werden. Während die Steuerung den Widerstand in der ersten Richtung in Abhängigkeit der Position und/oder der Lage des Kopfes einstellt, kann in jeder anderen Richtung der gleiche Widerstand oder ein anderer Widerstand verwendet werden, sofern durch einen oder mehrere Sensoren erkannt wird, dass der Träger der orthopädietechnischen Einrichtung die wenigstens zwei Bewegungselemente, die im vorliegenden Beispiel das Kugelgelenk bilden, in eine andere als die erste Richtung bewegen möchte.

Wird das wenigstens eine Positionierelement als Kugelgelenk ausgebildet gleiten in der Regel die wenigstens zwei Bewegungselemente entlang von kugelsegmentförmigen Flächen aufeinander ab. Über den Druck, mit dem die beiden Bewegungselemente aneinander angedrückt oder angepresst werden, lässt sich der Widerstand stufenlos einstellen.

Unabhängig von der Ausgestaltung des Positionierelementes kann jedoch eine Steuerung des Widerstandes auch darin bestehen, in Abhängigkeit der Position und/oder der Lage des Kopfes des Trägers der orthopädietechnischen Vorrichtung die Bewegung in die erste Richtung freizugeben oder zu blockieren, indem das Positionierelement arretiert wird.

In einer bevorzugten Ausgestaltung sind die wenigstens zwei Bewegungselemente in eine zweite Richtung relativ zueinander frei bewegbar, die der ersten Richtung entgegengesetzt ist.

Vorteilhafterweise handelt es sich bei der Steuerung um eine mechanische, elektronische oder mechatronische Steuerung. Insbesondere die Verwendung einer mechanischen Steuerung, die vorzugsweise völlig ohne elektronische Bauteile auskommt, ist von Vorteil, da keine externe Energieversorgung für elektronische Bauteile nötig ist und die Fehleranfälligkeit reduziert und gegebenenfalls dennoch nötige Reparaturen vereinfacht werden.

Vorteilhafterweise verfügt die Steuerung über wenigstens ein Kopfanlageelement, das derart angeordnet ist, dass sich seine Position relativ zu wenigstens einem weiteren Bauteil der Steuerung zumindest dann verändert, wenn der Kopf des Trägers in wenigstens einer vorbestimmten Richtung bewegt wird. Vorteilhafterweise ist das Kopfanlageelement ergonomisch an die Kopfform angepasst und bevorzugt so gepolstert, dass die Verwendung der orthopädietechnischen Vorrichtung bequem möglich ist. Das Kopfanlageelement kann vorzugsweise über ein Kraftübertragungselement, das vorzugsweis Druck- und Zugkräfte übertragen kann, mit einem weiteren Bauteil der Steuerung gekoppelt sein. Wird nun der Kopf in wenigstens einer vorbestimmten Richtung bewegt und so die Position und/oder die Lage des Kopfes verändert, wird diese Bewegung auf das wenigstens eine Kopfanlageelement und damit auch auf das Kraftübertragungselement übertragen und an die weiteren Bauteile der Steuerung weitergegeben. Dadurch können beispielsweise Federn gespannt oder entspannt oder Schalter betätigt werden, um den Widerstand, der einer Bewegung des Gelenkes in wenigstens einer Richtung entgegenwirkt, zu verändern, also zu erhöhen oder zu senken.

Das wenigstens eine Kopfanlageelement kann an verschiedensten Positionen am Kopf des Trägers angeordnet werden. Eine Anordnung am Hinterkopf des Trägers ist ebenso denkbar wie eine Positionierung am Kinn, der Stirn oder am Hals des Trägers, der in diesem Zusammenhang als zum Kopf gehörig angesehen wird.

Das Kraftübertragungselement kann beispielsweise in Form einer Stange vorhanden sein die gegebenenfalls in Richtung auf den Kopf des Trägers der orthopädietechnischen Einrichtung federbelastet ist. Das Kopfanlageelement ist vorteilhafterweise am Hinterkopf des Trägers der orthopädietechnischen Einrichtung angeordnet. Neigt dieser seinen Kopf zurück wird von einer Kaudal-Bewegung gesprochen, bei der das Kopfanlageelement nach unten verschoben, was über das Kraftübertragungselement an die weiteren Bauteile der Steuerung übertragen wird. Senkt die Person jedoch ihren Kopf, sorgt eine Vorrichtung, über die eine Vorspannung aufgebracht werden kann, beispielsweise eine Feder, dafür, dass das Kraftübertragungselement und damit auch das Kopfanlageelement dieser Bewegung des Kopfes folgt, sodass es immer zu einem Kontakt zwischen dem Kopfanlageelement und dem Kopf des Trägers der orthopädietechnischen Einrichtung kommt.

Alternativ kann durch das wenigstens eine Kraftübertragungselement auch lediglich eine Zugkraft übertragen werden. In diesem Fall kann beispielsweise durch eine Zugkraft ein Gelenk entsperrt oder verriegelt werden, indem beispielsweise eine Sperrklinke oder ein Sperrhebel mit einem Zahnrad oder einer anderen mit Zähnen versehenen Einrichtung in Eingriff oder außer Eingriff gebracht wird. Durch einen passiven Mechanismus, beispielsweise ein Feder, kann dies wieder rückgängig gemacht und die Sperrklinke oder der Sperrhebel in den ursprünglichen Zustand zurückversetzt werden.

Die Neigung des Kopfes relativ zur Horizontalen ist ein besonders bevorzugt verwendeter Steuerparameter, da einerseits eine entsprechende Bewegung des Kopfes leicht detektierbar und auch über mechanische Elemente übertragbar ist und zum anderen beispielsweise bei Arbeiten über Kopf die Bewegung des Kopfes in die entsprechende Richtung dem natürlichen Arbeitsauflauf und der dabei auftretenden natürlichen Bewegung entspricht. Soll eine Arbeit über Kopf ausgeführt werden, beispielsweise eine Montage oder das Streichen einer Decke, wird die entsprechende Person ohnehin ihren Kopf nach oben wenden, um mit den Augen der Arbeit ihrer Hände zu folgen. In diesem Fall wird beispielsweise eine Unterstützung von Oberarmstützelementen benötigt, um das Emporhalten der Hände möglichst zu vereinfachen. Ist hingegen die Arbeit über Kopf beendet, senkt der Träger orthopädietechnischen Einrichtung den Kopf und der Widerstand, der eine Bewegung des wenigstens einen Gelenkes in einer Richtung entgegensteht, kann wieder auch geändert werden. Dabei ist zu beachten, dass ein Widerstand der einer Bewegung des Gelenkes in einer Richtung entgegensteht eine Bewegung des Gelenkes in die entgegengesetzte Richtung gegebenenfalls unterstützt. Auch diese Ausführungsform wird im Rahmen der vorliegenden Erfindung unter einem Widerstand verstanden, der eine Bewegung des wenigstens einen Gelenkes in wenigstens einer Richtung entgegensteht.

Für einige Anwendungen ist jedoch die Neigung des Kopfes relativ zur Horizontalen kein geeigneter Steuerparameter. Dies betrifft beispielsweise Arbeiten, bei denen die Person auf dem Rücken liegend arbeitet, wie dies beispielsweise in Kraftfahrzeugwerkstätten vorkommt, wenn ein Mechaniker unter dem Auto liegt und arbeitet. In diesem Fall ist es von Vorteil, nicht einen Neigungswinkel des Kopfes relativ zur Horizontalen, sondern relativ zur Transversalebene des Trägers der Vorrichtung zu bestimmen. Die Horizontale wird in der Regel definiert als eine Ebene, die senkrecht auf dem Schwerefeld der Erde steht. Wenn eine Person aufrecht steht oder geht, fällt diese zumindest nahezu mit der Transversalebene des Körpers der Person überein. Insbesondere bei Anwendungen, bei denen dies nicht der Fall ist, ist es jedoch oftmals sinnvoll, einen Neigungswinkel des Kopfes relativ zur Transversalebene des Trägers zu definieren. In diesem Fall könnte beispielsweise ein Positionssensor oder Lagesensor am Rumpf des Trägers der Vorrichtung positioniert werden, der orts- und insbesondere drehfest mit dem Rumpf verbunden ist, sodass auf diese Weise die Position und Lage einer Transversalebene einfach bestimmt werden kann. Diese kann beispielsweise als Winkel zur Horizontalen angegeben werden. Wird gleichzeitig auch der Winkel beispielsweise des Kopfes zur Horizontalen gemessen, kann aus einer Kombination der beiden Ergebnisse auch ein Winkel des Kopfes relativ zur Transversalebene bestimmt werden.

Vorzugsweise blockiert die Steuerung eine Bewegung der Bewegungselemente in der ersten Richtung, wenn der Neigungswinkel des Kopfes relativ zur Horizontalen oder der Transversalebene einen vorbestimmten Grenzwert überschreitet. Dies ist insbesondere für eine Unterstützende orthopädietechnische Vorrichtung von Vorteil, die im bereits genannten Fall eines über Kopf arbeitenden Trägers der Vorrichtung von Vorteil ist. Wird der Kopf so weit angehoben, dass ein vorbestimmter Grenzwert, er beispielsweise ein vorbestimmter Winkel sein kann, überschritten wird, wird eine Bewegung der Bewegungselemente, die einem Absenken des Armes entspricht, blockiert. Der Träger der orthopädietechnischen Einrichtung muss dann keine Kraft mehr über die Schultern oder die Arme aufwenden, um die Arme in dieser ansonsten unbequemen Position zu halten.

Vorzugsweise ermöglicht die Steuerung die Bewegung des Gelenkes in die wenigstens eine Richtung, so lange der Neigungswinkel des Kopfes relativ zur Horizontalen und/oder einer Transversalebene abnimmt und/oder den vorbestimmten Grenzwert unterschreitet. Dieser Fall tritt ein, wenn die Person, die über Kopf arbeitet, den Blick und damit den Kopf wieder senkt. Vorzugsweise detektiert die Vorrichtung das Absenken des Kopfes und die Steuerung ermöglicht auch die Bewegung der Bewegungselemente wieder in die ursprünglich blockierte Richtung. Dies entspricht im beschriebenen Beispiel einem Absenken der Arme, das immer dann möglich sein soll, wenn auch der Kopf gesenkt wird. Wird der Kopf wieder angehoben oder die Senkbewegung des Kopfes beendet, blockiert die Steuerung wieder die Bewegung der Bewegungselemente in die entsprechende Richtung, so lange der Neigungswinkel des Kopfes relativ zur Horizontalen den vorbestimmten Grenzwert noch immer überschreitet. Anderenfalls bleibt die Bewegung frei möglich.

Selbstverständlich ist es auch möglich, die Bewegung der Bewegungselemente bei Überschreiten eines Messparameters wie beispielsweise des Neigungswinkels des Kopfes relativ zur Horizontalen oder einer Transversalebene, nicht vollständig zu blockieren, sondern beispielsweise durch das Aufbringen einer gegebenenfalls frei einzustellenden Kraft zu erschweren. Dies kann beispielsweise durch eine variierende, beispielsweise ansteigende oder absinkende Kraft oder durch einen frei wählbaren Kraftverlauf geschehen. Dieser kann für unterschiedliche Messparameter oder unterschiedliche Bereiche, in denen der Messwert des jeweiligen Messparameters liegt, auch durch unterschiedliche Kraftverläufe realisiert werden, die beispielsweise in einer dafür vorgesehenen elektronischen Datenspeichereinrichtung hinterlegt sein können. Selbstverständlich kann die Kraft auch über den gesamten Bereich des Messparameters oder Abschnittsweise konstant ausgebildet sein. Im beschriebenen Ausführungsbeispiel hieße das, die Arme in der Haltung über Kopf zu unterstützen, ein Absenken jedoch weiterhin zu ermöglichen. Die für diesen Fall aufgebrachte Kraft, die einer Bewegung der Bewegungselemente in die eine Richtung entgegensteht, kann vom jeweiligen Neigungswinkel abhängig gemacht werden, sodass sie beispielsweise zunimmt, je weiter der Träger der orthopädietechnischen Einrichtung den Kopf hebt. Auch hier sind mehrere Grenzwerte möglich, bei deren Überschreiten die Kraft beispielsweise ansteigt, wobei insbesondere bei Überschreiten des letzten Grenzwertes eine vollständige Arretierung, Sperrung und Blockierung des Positionierelementes möglich ist. Auch hier ist diese Sperrung sowohl in einer Richtung als auch in beide Richtungen des Positionierelementes möglich.

In einer bevorzugten Ausgestaltung verfügt die orthopädietechnische Vorrichtung über wenigstens zwei Positionierelemente und die Steuerung ist eingerichtet, einem Widerstand, der einer Bewegung der Bewegungselemente wenigstens eines der wenigstens zwei Positionierelemente entgegensteht, in Abhängigkeit einer Position und/oder Lage des Kopfes des Trägers der Vorrichtung einzustellen. Insbesondere wenn die orthopädietechnische Vorrichtung für beide obere Extremitäten ausgebildet ist, und somit auf beide Arme oder Teile beider Arme einen Effekt ausübt, ist diese Ausgestaltung von Vorteil.

Als vorteilhaft hat es sich dabei herausgestellt, wenn die Steuerung eingerichtet ist, den Widerstand für die wenigstens zwei Positionierelemente unabhängig voneinander einzustellen. Insbesondere in diesem Fall aber auch in allen andern Ausgestaltungen ist es von Vorteil, nicht nur oder gegebenenfalls gar nicht die Neigung des Kopfes relativ zur Horizontalen oder einer Transversalebene sondern beispielsweise auch eine Drehung des Kopfes nach rechts und links oder eine Verkippung des Kopfes um eine von dorsal nach ventral verlaufende Achse als Steuerparameter zu verwenden.

Vorzugsweise verfügt die Steuerung über wenigstens zwei Kopfanlageelemente, die derart angeordnet sind, dass sich ihre Position relativ zu wenigstens einem weiteren Bauteil der Steuerung zumindest dann verändert, wenn der Kopf des Trägers in wenigstens einer vorbestimmten Richtung bewegt wird. Wie auch bei der Ausgestaltung mit nur einem Kopfanlageelement kann sich die Position jedes der Kopfanlageelemente oder nur eines oder weniger der Kopfanlageelemente natürlich auch dann verändern, wenn der Kopf in mehrere Richtungen bewegt wird. Mit zwei unabhängigen Kopfanlageelementen ist es auf besonders einfache Weise auch mechanisch möglich, die Widerstände für wenigstens zwei Gelenke unabhängig voneinander einzustellen.

Vorteilhafterweise verfügt die Vorrichtung über wenigstens einen Sensor zum Detektieren der Position und/oder der Lage des Kopfes des Trägers und zum Senden von elektronischen Daten in Abhängigkeit der Detektierten Position und/oder Lage an die Steuerung. Zusätzlich oder anstelle von einer mechanischen Steuerung wird auf diese Weise eine elektronische oder mechatronische Steuerung gewährleistet, da die vom Sensor detektierte Position und/oder Lage des Kopfes durch entsprechende Signale an die Steuerung weitergegeben wird, die anschließend mechanische oder elektronisch dafür sorgt, dass der entsprechende Widerstand eingestellt wird. Dazu sendet sie beispielsweise Steuersignale an eine Einstelleinrichtung, die vorteilhafterweise direkt am Positionierelement angeordnet ist und als Reaktion auf die eingehenden Steuersignale einem Widerstand, der der Bewegung des entsprechenden Gelenkes in wenigstens einer Richtung entgegensteht, einstellt. Auch hier kann es von einer Veränderung der Kraft oder des Widerstandes bis hin zu einem völligen Verriegeln oder Entriegeln kommen. Auch dies ist selbstverständlich in Abhängigkeit der detektierten Position und/oder Lage des Kopfes möglich.

Der wenigstens eine Sensor ist vorteilhafterweise ein Lagesensor, ein Winkelsensor, ein Bewegungssensor oder ein Beschleunigungssensor, durch den folglich die Lage, die Position oder die Bewegung oder die Beschleunigung des Kopfes messbar ist. Es können auch ein oder mehrere Drucksensoren, Drehsensoren, Magnetfeldsensoren oder Abstandssensoren, beispielsweise für Ultraschall oder Nahfeldradar-Messungen verwendet werden. Insbesondere aus Sensorinformationen, also vom Sensor detektierten Positionen und/oder Lagen aber auch bei mechanischen Erfassungsvorrichtungen kann über einen Widerstand, der der Bewegung des Gelenks in wenigstens einer Richtung entgegensteht, also insbesondere eine auf das Gelenk und/oder auf wenigstens eines der gelenkig miteinander verbundenen Bauteile wirkenden Kraft auch die Gelenkposition selbst gesteuert und angepasst werden. So ist es beispielsweise möglich die Position oder Lage einer Prothesenhand allein mit dem Kopf zu steuern und so dafür zu sorgen, dass beispielsweise die Position der Hand einer Orientierung oder Drehrichtung des Kopfes folgt.

Der wenigstens eine Sensor kann in einer bevorzugten Ausgestaltung an einem speziell dafür vorgesehenen Kopfanlageelement befestigt werden. Alternativ oder zusätzlich dazu ist es auch möglich, wenigstens einen Sensor an einem Gegenstand zu befestigen, der ohnehin am Kopf des Trägers der orthopädietechnischen Vorrichtung getragen wird. Dies kann beispielsweise eine Brille, ein Stirnband oder beispielsweise im Arbeitsumfeld des Trägers auch ein Helm oder eine andere Vorrichtung sein.

Zusätzlich zu der Steuerung des Widerstandes in Abhängigkeit von der Position und/oder der Lage eines Kopfes kann als weiterer Steuerparameter beispielsweise auch eine Beschleunigung des Kopfes verwendet werden. Dazu ist es naturgemäß von Vorteil, wenn der wenigstens eine Sensor zumindest einen Beschleunigungssensor beinhaltet. Übersteigt die gemessene Beschleunigung beispielsweise einen vorbestimmten Schwellwert, der vorzugsweise einstellbar ausgebildet ist und in einer besonders bevorzugten Ausgestaltung vom Träger der orthopädietechnischen Vorrichtung selbst eingestellt werden kann, so wird dies als reflexartige Bewegung gewertet, die beispielsweise eine sofortige Freigabe der Bewegung in die erste Richtung erlaubt.

Insbesondere bei einer mechanischen Detektion der Kopfposition über wenigstens ein Kopfanlageelement ist es selbstverständlich auch möglich, die Mechanik so auszubilden, dass auf ein Kraftübertragungselement ein Zug ausgeübt wird, wenn der Träger der orthopädietechnischen Einrichtung den Kopf hebt, ihn also nach hinten neigt. Bei der umgekehrten Bewegung, also dem Neigen des Kopfes nach vorne, also dem Senken des Kopfes, wird die Spannung auf die Kabel oder den ähnlichen Mechanismus, der als Kraftübertragungselement verwendet wird, reduziert und diese Veränderung in der Spannung zur Steuerung des Widerstandes, beispielsweise zum Lösen einer Verriegelung des Gelenkes, verwendet.

Mit Hilfe der beiliegenden Figuren wird nachfolgend ein Ausführungsbeispiel der vorliegenden Erfindung näher erläutert: Es zeigt:
- Figur 1a und 1b -: die schematische Darstellung einer Person, die eine orthopädietechnische Vorrichtung gemäß einem ersten Ausführungsbeispiel der vorliegenden Erfindung trägt,
- Figur 2 -: ein vergrößertes Detail und
- Figur 3 -: ein weiteres Ausführungsbeispiel.

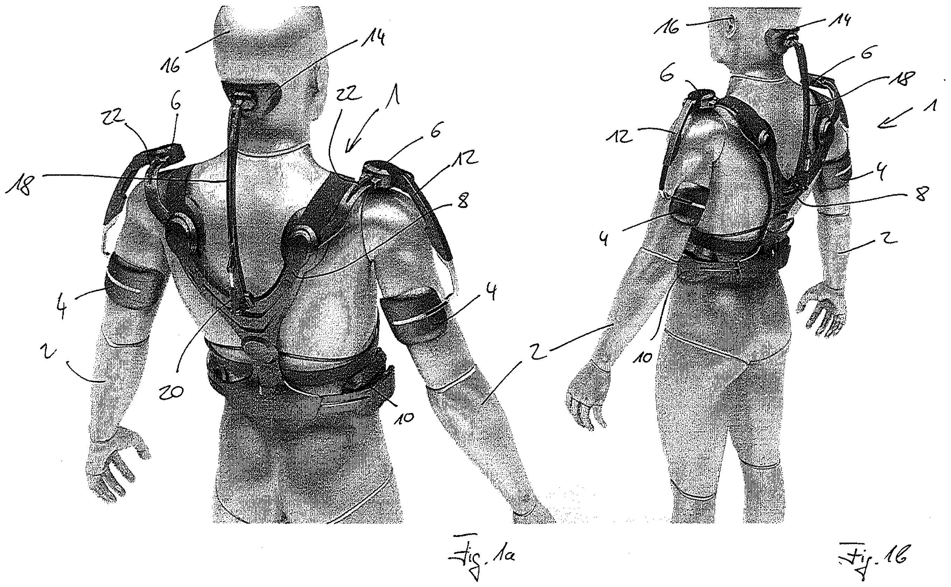

Figuren 1a und 1b zeigen eine orthopädietechnische Vorrichtung 1 gemäß einem ersten Ausführungsbeispiel der vorliegenden Erfindung. Es handelt sich um eine Stützvorrichtung, mit der die Arme 2 des Trägers der Vorrichtung 1 abgestützt werden können. Dafür verfügt die Vorrichtung über zwei Armauflagen 4, die gelenkig über Gelenke 6, die in diesem Fall die Positionierelemente bilden, an einem Rückenelement 8 angeordnet sind und beispielsweise als Polster, Armschelle, Manschette, Armführung oder Armaufnahme ausgebildet sein können. Dieses verfügt über Stützelemente 10, über die die orthopädietechnische Vorrichtung 1 am Rücken und am Torso des Trägers abgestützt wird. Das Gelenk 6 erlaubt eine Bewegung eines Verbindungselementes 12 relativ zum Rückelement 8 so, dass jeder Bewegung des Arms 2 gefolgt werden kann, sodass die Armauflage 4 immer am Arm 2 anliegt.

Die orthopädietechnische Vorrichtung 1 verfügt zudem über ein Kopfanlageelement 14, das am Kopf 16 des Trägers anliegt und einer Bewegung des Kopfes folgt. Über ein Kraftübertragungselement 18 wird jede Bewegung des Kopfes, die auf das Kopfanlageelement 14 übertragen wird, an die Steuerung übergeben. Im gezeigten Ausführungsbeispiel verfügt die Steuerung über einen Zylinder 20, der bei einer Bewegung des Kopfes komprimiert oder auseinandergezogen wird.

Neigt der Träger der orthopädietechnischen Vorrichtung 1 wie in den Figuren 1a und 1b gezeigt den Kopf 16 nach hinten, wird ein Stift in den Zylinder hineingedrückt, sodass ein Verriegelungselement betätigt wird. Man erkennt einen Bowdenzug 22, die in die einzelnen Gelenke 6 hineinführt. Dadurch kann das durch den Zylinder 20 übertragene Steuersignal in das Gelenk 6 übertragen werden, wo es dafür sorgt, den Widerstand, der einer Bewegung des Gelenkes 6 in eine bestimmte Richtung entgegensteht, anzupassen und das Gelenk 6 gegebenenfalls vollständig zu verriegeln.

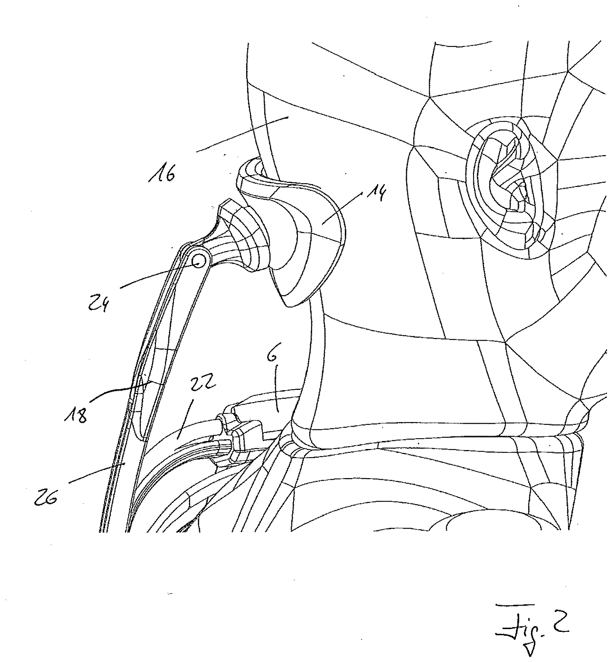

Figur 2 zeigt einen vergrößerten Ausschnitt aus einer derartigen Vorrichtung. Man erkennt am Kopf 16 des Trägers des Kopfanlageelementes 14 dass es ergonomisch geformt und gepolstert ist. Über ein Gelenk 24 ist es an einer Stützstruktur 26 befestigt, in der das eigentliche Kraftübertragungselement 18 verläuft. Dieses ist am in Figur 2 nicht gezeigten unteren Ende mit dem Kolben des Zylinders 20 verbunden, sodass eine Verschwenkung des Kopfanlageelementes 14 um die Gelenkachse des Gelenks 24 herum eine Bewegung des Kolbens innerhalb des Zylinders 20 zur Folge hat, wodurch die Bewegung detektiert und in ein Steuersignal umgesetzt wird. Für gegebenenfalls vom Kopf 16 des Trägers der Vorrichtung 1 durchgeführte Bewegungen, die nicht zu einer Verschwenkung des Kopfanlageelementes 14 um die Gelenkachse des Gelenks 24 führen, kann beispielsweise am Kopfanlageelement 14 ein Sensor oder mehrere Sensoren angeordnet werden, die derartige Bewegungen detektieren und beispielsweise in elektronische Steuersignale umsetzen. In Figur 2 ist zudem das Gelenk 6 mit dem entsprechenden Bowdenzug 22 zu erkennen.

Eine Ver- und Entriegelungsvorrichtung, wie sie in allen hier beschriebenen Ausführungsformen der Erfindung verwendet werden kann, besteht in einer besonders einfachen Ausgestaltung aus einer Sperrklinke, die durch eine Bewegung des Kopfes beispielsweise über ein mechanisches Kraftübertragungselement in bzw. außer Eingriff mit einer Zahnstange oder einem Zahnrad gebracht wird und so eine Bewegung der beiden Bewegungselemente des Positionierelementes in der zweiten Richtung erlaubt, in der ersten Richtung jedoch unterbindet, wenn die Klinke mit dem Zahnrad oder der Zahnstange in Eingriff steht. Wir sie außer Eingriff gebracht, ist die Bewegung in beide Richtungen wieder möglich.

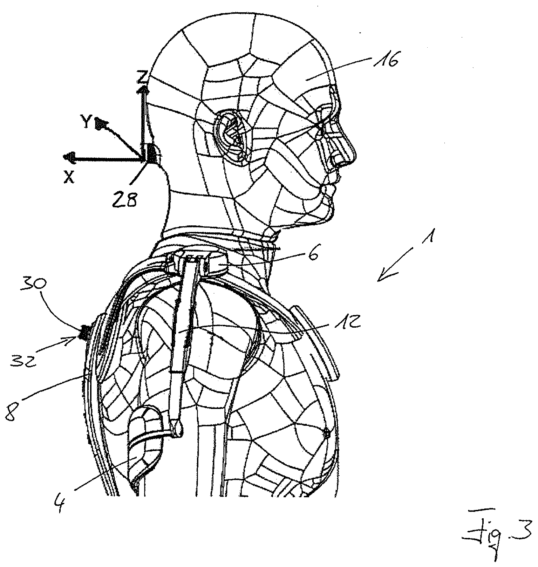

Figur 3 zeigt ein weiteres Ausführungsbeispiel der vorliegenden Erfindung. Auch hier verfügt die orthopädietechnische Vorrichtung ein über ein Gelenk 6, das an dem Rückenelement 8 angeordnet ist und das über das Verbindungselement 12 mit der Armauflage 4 in Verbindung steht. Anders als bei den bisher gezeigten Ausführungsbeispielen ist am Kopf 16 ein Sensor 28 angeordnet, der beispielsweise die Lage des Kopfes 16 ermittelt und über eine drahtlose Verbindung zu einem Empfänger 30 übermittelt. Aus den von dem Sensor 28 übermittelten Daten kann eine nicht gezeigte elektronische Steuerung Steuersignale erzeugen, um das Gelenk 6 zu steuern.

Der Sensor 28 kann dabei in eine Kopfstütze integriert oder direkt am Kopf getragen werden und beispielsweise dessen räumliche Orientierung erfassen. In Abhängigkeit dieser Orientierung des Kopfes wird das Gelenk 6 bei einer Drehung um die wie in Figur 3 gezeigte Y-Achse verriegelt oder entriegelt. Dieses mechatronische Gelenk 6 kann so ausgelegt sein, dass die Verriegelung erst oberhalb einer bestimmten Position des Gelenkes wirksam wird. Dabei wird eine weitere Bewegung der beiden Bewegungselemente des Gelenks 6 in die erste Richtung verhindert, eine Bewegung in die zweite Richtung bleibt möglich. Zur Bestimmung der relativen Lage des Kopfes 16 am Rumpf des Trägers kann ein weiterer Sensor 32 am Rumpf angeordnet sein, um eine relative Lage des Kopfes zum Rumpf zu ermitteln und so beispielsweise einen Winkel relativ zu einer Transversalebene als Steuerparameter verwenden zu können. Dies ist beispielsweise dann notwendig, wenn die Schwerpunkte des Kopfes 16 und des Rumpfes nicht im Lot sind. Dies ist beispielsweise bei einer gebeugten Haltung der Fall, in der die Verriegelungsposition des Gelenks 6 angepasst werden muss. Alternativ können die Verriegelungspositionen auch über unterschiedliche Programme gesteuert werden, die auf unterschiedliche Arbeitshaltungen des Trägers abgestimmt sind. Alternativ oder zusätzlich dazu kann auch beispielsweise die Lage des Sensors 28 je nach Arbeitshaltung justiert werden oder eine Ruhelage oder Nullposition kann eingestellt und kalibriert werden, um das Gelenk entsprechend früher oder später zu sperren.

### Bezugszeichenliste

- 1: orthopädietechnische Vorrichtung
- 2: Arm
- 4: Armauflage
- 6: Gelenk
- 8: Rückenelement
- 10: Stützelement
- 12: Verbindungselement
- 14: Kopfanlageelement
- 16: Kopf
- 18: Kraftübertragungselement
- 20: Zylinder
- 22: Bowdenzug
- 24: Gelenk
- 26: Stützstruktur
- 28: Sensor
- 30: Empfänger
- 32: weiterer Sensor

## Patentansprüche

1. Orthopädietechnische Vorrichtung (1) für wenigstens eine obere Extremität (2), wobei die Vorrichtung (1) wenigstens ein Positionierelement (6) aufweist, das wenigstens zwei Bewegungselemente aufweist, die relativ zueinander in eine erste Richtung bewegbar sind, wobei einer Bewegung der Bewegungselemente relativ zueinander in eine erste Richtung ein Widerstand entgegensteht,
**dadurch gekennzeichnet, dass**
- die Vorrichtung (1) eine Steuerung aufweist, die eingerichtet ist den Widerstand in Abhängigkeit der Position und/oder der Lage des Kopfes (16) des Trägers der Vorrichtung (1) einzustellen.

2. Orthopädietechnische Vorrichtung (1) nach Anspruch 1, **dadurch gekennzeichnet, dass** die Bewegungselemente relativ zueinander in eine zweite Richtung frei bewegbar sind, die der ersten Richtung entgegengesetzt ist.

3. Orthopädietechnische Vorrichtung (1) nach Anspruch 1 oder 2, **dadurch gekennzeichnet, dass** die Steuerung eine mechanische, elektronische oder mechatronische Steuerung ist.

4. Orthopädietechnische Vorrichtung (1) nach Anspruch 3, **dadurch gekennzeichnet, dass** die Steuerung wenigstens ein Kopfanlageelement (14) aufweist, das derart angeordnet ist, dass sich seine Position relativ zu wenigstens einem weiteren Bauteil der Steuerung zumindest dann verändert, wenn der Kopf (16) des Trägers in wenigstens einer vorbestimmten Richtung bewegt wird.

5. Orthopädietechnische Vorrichtung (1) nach einem der vorstehenden Ansprüche, **dadurch gekennzeichnet, dass** die Steuerung eine Bewegung des Positionierelementes (6) in wenigstens eine Richtung blockiert, wenn ein Neigungswinkel des Kopfes (16) relativ zur Transversalebene einen vorbestimmten Grenzwert überschreitet.

6. Orthopädietechnische Vorrichtung (1) nach Anspruch 5, **dadurch gekennzeichnet, dass** die Steuerung die Bewegung des Positionierelementes (6) in die erste Richtung ermöglicht, solange der Neigungswinkel des Kopfes (16) relativ zur Transversalebene abnimmt und/oder den vorbestimmten Grenzwert unterschreitet.

7. Orthopädietechnische Vorrichtung (1) nach einem der vorstehenden Ansprüche, **dadurch gekennzeichnet, dass** die Vorrichtung (1) wenigstens zwei Positionierelemente (6) aufweist, wobei die Steuerung vorzugsweise eingerichtet ist den Widerstand, der einer Bewegung der Bewegungselemente in die erste Richtung relativ zueinander entgegensteht, für die wenigstens zwei Positionierelemente (6) unabhängig voneinander einzustellen.

8. Orthopädietechnische Vorrichtung (1) nach einem der vorstehenden Ansprüche, **dadurch gekennzeichnet, dass** die Steuerung wenigstens zwei Kopfanlageelemente (14) aufweist, die derart angeordnet sind, dass sich ihre Position relativ zu wenigstens einem weiteren Bauteil der Steuerung zumindest dann verändert, wenn der Kopf (16) des Trägers in wenigstens einer vorbestimmten Richtung bewegt wird.

9. Orthopädietechnische Vorrichtung (1) nach einem der vorstehenden Ansprüche, **dadurch gekennzeichnet, dass** die Vorrichtung (1) wenigstens einen Sensor zum Detektieren der Position und/oder der Lage des Kopfes (16) des Trägers und zum Senden von elektronischen Signalen in Abhängigkeit der detektierten Position und/oder Lage an die Steuerung aufweist.

10. Orthopädietechnische Vorrichtung (1) nach Anspruch 9, **dadurch gekennzeichnet, dass** der wenigstens eine Sensor ein Lagesensor, ein Winkelsensor, ein Bewegungssensor oder ein Beschleunigungssensor ist.

## Claims

1. Orthopedic device (1) for at least one upper extremity (2), said device (1) comprising at least one positioning element (6) that has at least two movement elements which can be moved one relative to another in a first direction, wherein a movement of said movement elements relative to one another in a first direction encounters a resistance **characterised in that**
- said device (1) comprises a control unit configured to adjust this resistance depending on the position and/or the orientation of the head (16) of the wearer of the device (1).

2. Orthopedic device (1) according to claim 1, **characterised in that** the movement elements can be freely moved relative to one another in a second direction, which is in the opposite direction to the first.

3. Orthopedic device (1) according to claim 1 or 2, **characterised in that** the control unit is a mechanic, electronic or mechatronic control unit.

4. Orthopedic device (1) according to claim 3, **characterised in that** the control unit has at least one head support element (14) which is arranged in such a way that its position changes relative to at least one further component of the control unit if the head (16) of the wearer is moved in at least one pre-defined direction.

5. Orthopedic device (1) according to one of the above claims, **characterised in that** the control unit blocks a movement of the positioning element (6) in at least one direction if an angle of inclination of the head (16) relative to the transverse plane exceeds a pre-defined limit.

6. Orthopedic device (1) according to claim 5, **characterised in that** the control unit enables a movement of the positioning element (6) in the first direction as long as the angle of inclination of the head (16) relative to the transverse place decreases and/or does not reach the pre-defined limit.

7. Orthopedic device (1) according to one of the above claims, **characterised in that** the device (1) comprises at least two positioning elements (6), the control unit preferably being configured to adjust the resistance encountered by a movement of the movement elements in the first direction relative to one another for the at least two positioning elements (6) independently of one another.

8. Orthopedic device (1) according to one of the above claims, **characterised in that** the control unit has at least two head support elements (14) which are arranged in such a way that their position changes relative to at least one further component of the control unit if the head (16) of the wearer is moved in at least one pre-defined direction.

9. Orthopedic device (1) according to one of the above claims, **characterised in that** the device (1) has at least one sensor for detecting the position and/or orientation of the head (16) of the wearer and for sending electronic signals to the control unit depending on the detected position and/or orientation.

10. Orthopedic device (1) according to claim 9, **characterised in that** the at least one sensor is an orientation sensor, an angle sensor, a movement sensor or an acceleration sensor.

## Revendications

1. Dispositif orthopédique (1) pour au moins une extrémité supérieure (2), le dispositif (1) comprenant au moins un élément de positionnement (6) qui présente au moins deux éléments de mouvement qui sont mobiles l'un par rapport à l'autre dans une première direction, une résistance étant opposée à un mouvement des éléments de mouvement l'un par rapport à l'autre dans une première direction,
**caractérisé en ce que**
- le dispositif (1) comprend une commande qui est conçue pour régler la résistance en fonction de la position et/ou de la posture de la tête (16) de l'utilisateur du dispositif (1).

2. Dispositif orthopédique (1) selon la revendication 1,
**caractérisé en ce que**
les éléments de mouvement sont librement mobiles l'un par rapport à l'autre dans une seconde direction opposée à la première direction.

3. Dispositif orthopédique (1) selon la revendication 1 ou 2,
**caractérisé en ce que**
la commande est une commande mécanique, électronique ou mécatronique.

4. Dispositif orthopédique (1) selon la revendication 3,
**caractérisé en ce que**
la commande comprend au moins un élément d'appui de tête (14) qui est agencé de telle sorte que sa position par rapport à au moins un autre composant de la commande se modifie au moins lorsque la tête (16) de l'utilisateur est déplacée dans au moins une direction prédéterminée.

5. Dispositif orthopédique (1) selon l'une des revendications précédentes,
**caractérisé en ce que**
la commande bloque un mouvement de l'élément de positionnement (6) dans au moins une direction lorsqu'un angle d'inclinaison de la tête (16) par rapport au plan transversal dépasse une valeur limite prédéterminée.

6. Dispositif orthopédique (1) selon la revendication 5,
**caractérisé en ce que**
la commande permet le mouvement de l'élément de positionnement (6) dans la première direction tant que l'angle d'inclinaison de la tête (16) par rapport au plan transversal diminue et/ou passe au-dessous de la valeur limite prédéterminée.

7. Dispositif orthopédique (1) selon l'une des revendications précédentes,
**caractérisé en ce que**
le dispositif (1) comprend au moins deux éléments de positionnement (6), la commande étant de préférence conçue pour régler la résistance, opposée à un mouvement des éléments de mouvement l'un par rapport à l'autre dans la première direction, pour lesdits au moins deux éléments de positionnement (6) indépendamment l'un de l'autre.

8. Dispositif orthopédique (1) selon l'une des revendications précédentes,
**caractérisé en ce que**
la commande comprend au moins deux éléments d'appui de tête (14) qui sont agencés de telle sorte que leur position par rapport audit au moins un autre composant de la commande se modifie au moins lorsque la tête (16) de l'utilisateur est déplacée dans au moins une direction prédéterminée.

9. Dispositif orthopédique (1) selon l'une des revendications précédentes,
**caractérisé en ce que**
le dispositif (1) comprend au moins un capteur pour détecter la position et/ou la posture de la tête (16) de l'utilisateur et pour envoyer à la commande des signaux électroniques en fonction de la position et/ou de la posture détectée.

10. Dispositif orthopédique (1) selon la revendication 9,
**caractérisé en ce que**
ledit au moins un capteur est un capteur de position, un capteur d'angle, un capteur de mouvement ou un capteur d'accélération.
